# EUROPEAN PATENT SPECIFICATION

(11) **EP 3 552 590 B1**
(45) Date of publication and mention of the grant of the patent: **20.04.2022**
(21) Application number: 18188917.1
(22) Date of filing: 14.08.2018
(51) Int. Cl.: A61F 13/495, A61F 13/494

(54) **ANTI-IRRITANT DIAPER**
IRRITATIONSFREIE WINDEL
COUCHE-CULOTTE ANTI-IRRITANT

(30) Priority: 10.04.2018 US 201815949094
(43) Date of publication of application: 16.10.2019
(73) Proprietor: Ben Natan, Chaim, 6084000 Kfar Chabad (IL); Ben Natan, Amichai, 1323667 Tzfat (IL)
(72) Inventor: Ben Natan, Chaim, 6084000 Kfar Chabad (IL); Ben Natan, Amichai, 1323667 Tzfat (IL)
(74) Representative: Kancelaria Eupatent.pl Sp. z.o.o

(56) References cited:
- WO-A2-01/19411
- WO-A2-2014/009959
- US-A1- 2006 004 340
- US-A1- 2007 239 132
- US-B1- 7 772 455

## Description

### FIELD OF THE INVENTION

The present invention relates to diapers, in particular to diapers constructed for protection from skin irritation.

### BACKGROUND OF THE INVENTION

Use of disposable diapers has provided a convenient and sanitary solution for parents of infants and care-givers to the elderly. However, there are some disadvantages as well. First of all, disposable diapers contain chemicals which can irritate a baby's skin, causing diaper rash and worse.

Secondly, approximately 90-95% of American babies use 27.4 billion single-use, plastic diapers every year. This generates 7.6 billion pounds of garbage each year. It is estimated to require between 250 and 500 years for a single disposable diaper to break down in a landfill.

And lastly, disposable diapers are expensive, estimated to cost approximately $2000 per child in 2.5 years.

The solution for these problems is using cloth diapers instead. Cloth diapers are used over and over before heading to the landfill, and they require about 5 months to break down. They are less costly and although they cost more upfront, they save costs considerably in the long run.

However, diaper rash caused by the contact of bodily waste with the skin is still a problem for disposable and cloth diapers alike. It would be beneficial to come up with a solution for this problem which is inflicted upon babies and adults wearing diapers.

The contact between solid or semi-solid waste (hereinafter "solid waste") waste and the skin can cause irritation and even pain, especially if the diaper-wearer has sensitivity to certain types of food. Also, care-givers suffer from discomfort and repulsion caused by changing soiled diapers and having to scrub the waste off the patient's skin with soap and water. In addition, solid waste often leakes from the diaper onto the legs and clothes.

A solution for the above-mentioned problems has been proposed by the Applicant in US Patent No. 6,464,673 and US Patent Application No. 14/413,010, both disclosing a diaper having a solid waste containment means.

WO2014009959 discloses an anti-irritant disposable diaper, comprising a semi- rigid element defining and maintaining a gap extending along a user contour and a waste containment means integrally formed within a crotch area and disposed below the gap for receiving excreted waste.

The present invention is an improvement of the above-mentioned inventions.

### SUMMARY OF THE INVENTION

The present invention overcomes the problems associated with the prior art by providing an anti-irradiant diaper construction which contains solid waste and distances the surface of the absorbent material from the skin of the user.

In embodiments of the present invention there is provided an anti-irritant diaper according to the appended claims.

Additional features and advantages will become apparent from the following drawings and description.

The pocket forms a space between the diaper and the wearer's skin so as to prevent the waste from coming in contact with the skin of the wearer, so as not to cause rashes. This also makes cleaning the diaper-wearer easier, during the diaper changing process, since the skin remains relatively clean. The flexible band may be bent by the movement of the wearer, for example when sitting down, and because the band is resilient, it returns to its original shape once the diaper-wearer returns to an upright position.

According to another preferred embodiment of the present invention, the diaper may be disposable or may be made of cloth for multiple use.

The embodiment of the disposable diaper also helps reduce the amount of waste, because the diaper can be changed less frequently than regular known diapers.

According to yet another preferred embodiment of the present invention, the flexible band may be removed from the diaper and re-inserted into a new one.

The present invention is useful for both infants and the elderly who cannot control their bowel movements. The diaper allows the diaper-wearer to be more mobile, since the solid waste is contained inside the pocket of the diaper and so, does not allow it to escape onto the legs and clothes, as often occurs when using a regular diaper. The contact between the waste and the skin is reduced to a minimum, if any, so that any feeling of discomfort is decreased. It is especially useful in places and situations where it is difficult to change the diaper, and the wearer is forced to remain with the soiled diaper for an extended period of time which by then, typically, would cause irritation to the skin.

The present invention is also especially useful for elderly patients in a nursing home, who are treated by care-givers whom also change their diapers. The process of changing diapers causes repulsion and disgust on behalf of the changer. The inventive diaper will minimize the care-giver's exposure to the solid waste during changing, because the waste will be safely contained inside the dedicated pocket.

The inventive diaper may also reduce the number of staff needed to care for the patients, since a great deal of time and work put into cleaning the patients, can be saved.

In yet another embodiment of the present invention there is provided a flexible band that is attached to the diaper but is inserted internally to the diaper, via a designated sleeve so it is not visible. The designated sleeve maintains the flexible band in its position, thereby maintaining the shape of the pocket.

In an additional embodiment of the present invention there is provided an external sleeve, designated for the insertion of the resilient flexible band.

In another additional embodiment of the present invention there is provided a resilient flexible band disposed transversely to the diaper, stretching from the right cuff to the left cuff.

In yet another preferred embodiment of the present invention there is provided a removable transverse one-directional flexible partition, situated above the pocket. The solid waste passes through the partition and into the pocket, and then it provides a cover over the waste to that the waste does not touch the skin. The flexible partition is made of a pair of dividers which are segmented into a plurality of flaps for preventing from the dividers to resist the weight of the waste. After the waste passes through the dividers, due to their flexibility, they return to their original position so that they function as a cover over the waste and therefore serve as a partition between the waste and the skin.

The removable transverse one-directional flexible partition may be disposable for one use only, or may be washed for multiple use.

In yet another embodiment of the present invention there is provided a cover for the pocket area, which can be pulled over the pocket by pulling a string external to the diaper, so that the solid waste captured in the pocket will not touch the skin. The cover may be spread by pulling the string on the opposite side to expose the pocket in the case of re-use by the diaper wearer. This feature is especially suitable for the elderly, who can spread and fold the cover themselves, more than once if needed, taking into consideration the diaper's capacity limit.

Additional features and advantages will become apparent from the following drawings and description.

### BRIEF DESCRIPTION OF THE DRAWINGS

For a better understanding of the invention with regard to the embodiments thereof, reference is made to the accompanying drawings, in which like numerals designate corresponding elements or sections throughout and in which:
Fig. 1 shows a back bottom perspective view of the diaper of the preferred embodiment of the present invention having a flexible band disposed externally to the diaper;
Fig. 2 shows a front bottom perspective view of the diaper of Fig. 1;
Fig. 3 shows a side perspective view of the diaper of Fig. 1 showing a flexible band being inserted into the diaper;
Fig. 4 shows the diaper having the flexible band disposed externally via a designated sleeve;
Fig. 5 shows the diaper having a transverse flexible band;
Fig. 6 shows a back bottom view of the diaper having the flexible band internally disposed;
Fig. 7 shows the diaper of Fig. 5 showing the flexible band being inserted;
Fig. 8 shows a top perspective view of the diaper of Fig. 1 showing a one-way flexible partition;
Fig. 9 shows the one-way flexible partition of Fig. 8, illustrating its movement towards the pocket; and
Figs. 10-12 show another preferred embodiment of the diaper having a moveable cover sheet.

### DETAILED DESCRIPTION OF THE EMBODIMENTS

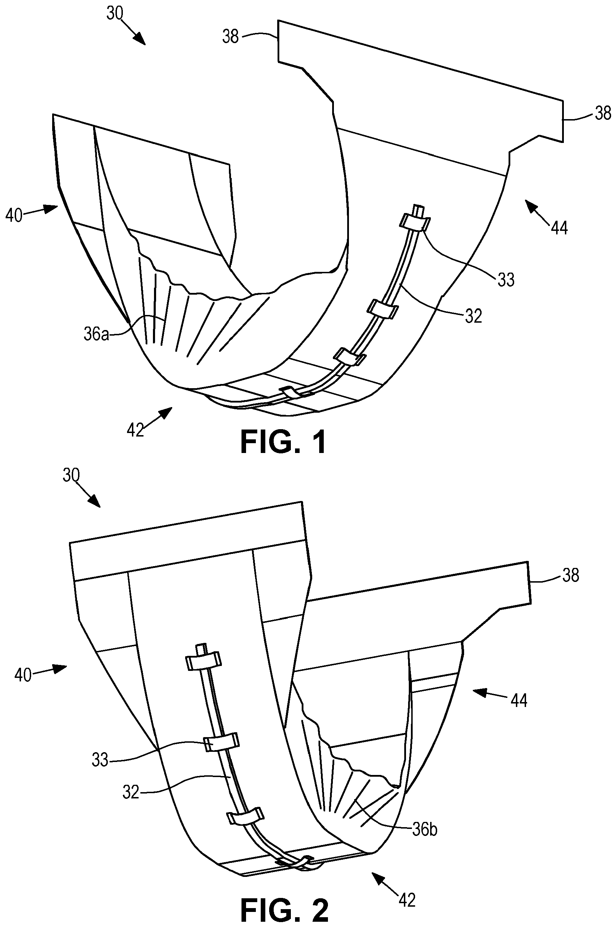

Referring now to Fig. 1, there is shown diaper 30 constructed in accordance with the principles of the present invention. Diaper 30 is constructed of a front area 40, a middle area 42 and a rear area 44. The rear area 44 has two fasteners 38, one on each side (as in conventional diapers) for closing the diaper. Middle area 42 comprises side cuffs 36a, 36b, one on each side (as in conventional diapers).

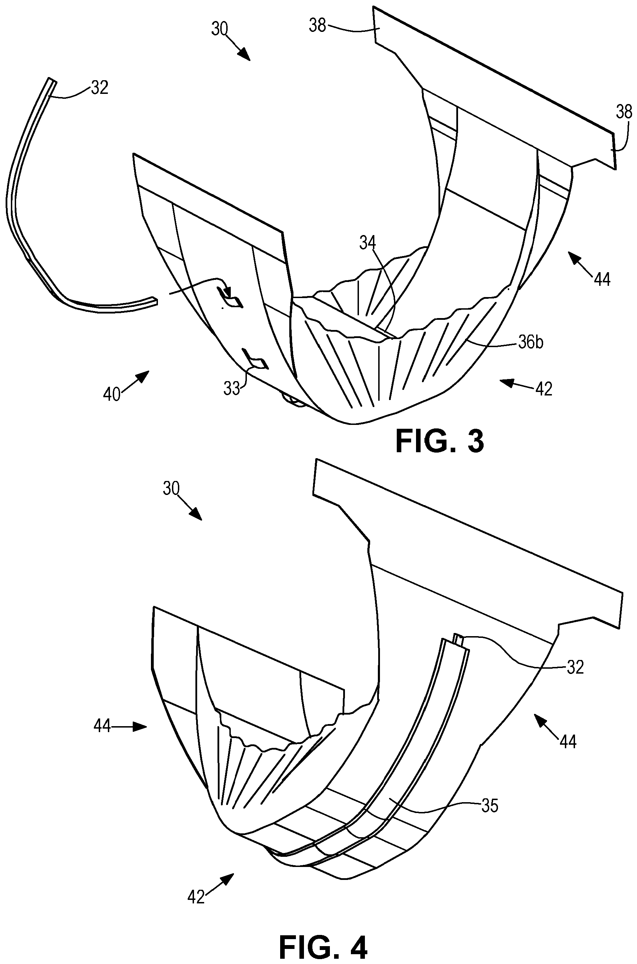

A resilient, flexible band 32 is removably disposed longitudinally and centrally along the outer side of diaper 30, from front area 40, continuing onto middle area 42 to rear area 44. Flexible band 32 is attached to diaper 30 by stick-on tabs 33. Flexible band 32 defines a pocket 34 (shown in Fig. 3) formed in middle area 42, and maintains its shape. Solid waste released from the wearer of diaper 30 falls into pocket 34 and is maintained there, thereby being prevented from leaking out of diaper 30. Pocket 34 is important for creating a gap between the waste and the user's skin to avoid irritation. Flexible band 32 is fabricated from a material that is rigid enough to maintain the form of pocket 34 while being resilient so that in the event the wearer sits down and thereby causes band 32 to bend, the band 32 will return to its original shape once the wearer stands in an upright position, thereby maintaining the shape of pocket 34. The waste stored in pocket 34 is safely kept away from the skin, thereby preventing irritation.

In the event of diaper 30 becoming full of urine so that it becomes heavy, flexible band 32 retains diaper 30 close to the wearer's body and does not allow diaper 30 to hang low between the legs.

Flexible band 32 may be removed from diaper 30 and placed on another diaper 32, so that there is a need for only one band 32 which can be re-used for multiple diaper 30 uses. Diaper 30 may be a multiple-use cloth diaper or a disposable type. A multi-use diaper 30 helps reduce the amount of waste caused by the wide usage of disposable diapers. Disposable diaper 30 also helps reduce the amount of waste, because the diaper 30 can be changed less frequently than regular known diapers.

The size of diaper 30 may be adapted to fit various sizes of wearers, from new-born babies to the elderly, and so the dimensions of the flexible band 32 are varied to fit these different-sized diapers 30, in order to form a suitable pocket 34 in the middle area 42.

Referring now to Fig. 2 there is shown a front bottom perspective view of diaper 30, showing flexible band 32 extending until front area 40.

Referring now to Fig. 3 there is shown band 32 removed from diaper 30. If diaper 30 is made of cloth, it can then be washed for re-use. After diaper 30 is cleaned, band 32 may be inserted again.

If diaper 30 is disposable, it is discarded and band 32 may be inserted into a new diaper 30.

Referring now to Fig. 4 there is shown diaper 30 having flexible band 32 inserted into a designated sleeve 35 situated along the outer side of diaper 30. Sleeve 35 allows for flexible band 32 to be inserted into it and pulled out of according to need. Sleeve 35 positions flexible band 32 along the center of diaper 30 and maintains it there so that the movement of the wearer does not affect the position of flexible band 32, thus maintaining the shape of pocket 34.

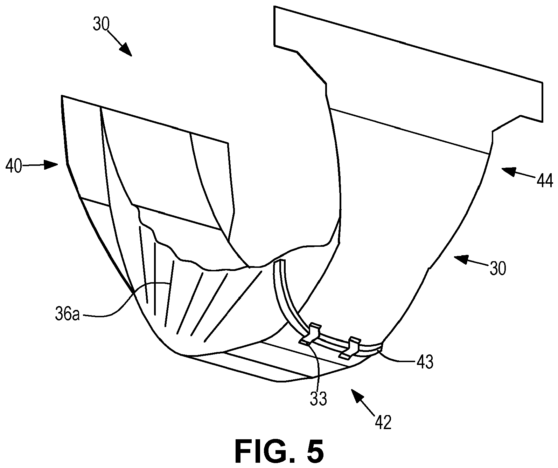

Referring now to Fig. 5 there is shown another embodiment of diaper 30 having a transverse flexible band 43, replacing flexible band 32, stretching from the end of cuff 36a to the end of cuff 36b. Transverse flexible band 43 defines a pocket 34 (see Fig. 3) formed in middle area 42.

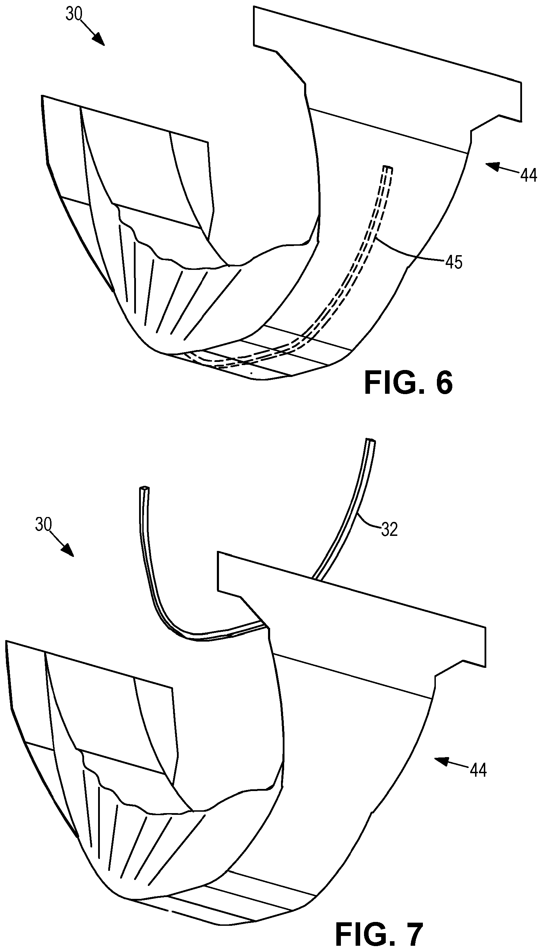

Referring now to Figs. 6 and 7 there is shown diaper 30 having a designated internal sleeve 45 for inserting flexible band 32. Flexible band 32 is not visible once it is inserted into sleeve 45. Sleeve 45 positions flexible band 32 along the center of diaper 30 and maintains it there so that the movement of the wearer does not affect the position of flexible band 32, thus maintaining the shape of pocket 34. Flexible band 32 may be pulled out and released from internal sleeve 45, as shown in Fig. 7, when diaper 30 is soiled and needs to be washed in the case of a cloth diaper or discarded in the case of a disposable diaper. Flexible band 32 may be re-used and inserted into another diaper 30 having internal sleeve 45.

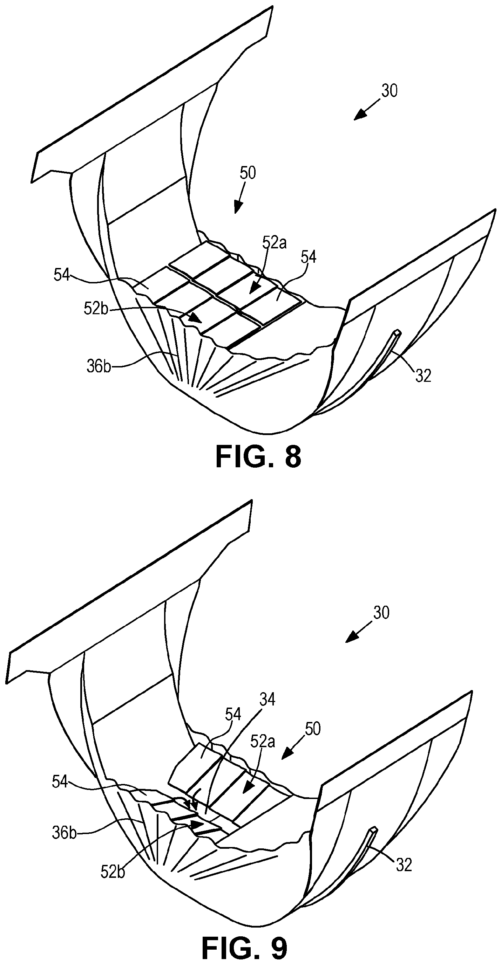

Referring now to Figs. 8 and 9 there is shown diaper 30 having flexible band 32 disposed on the outer side of diaper 30. A flexible partition 50 is provided to prevent the waste in pocket 34 from contacting the wearer's skin, thereby preventing skin irritation. The removable flexible partition 50 is made of a pair of dividers 52a and 52b, situated above pocket 34, each attached to leg cuff 36a and 36b, respectively. Each divider 52a-b is segmented into a plurality of flexible flaps 54 for preventing dividers 52a-b from resisting the weight of the waste. Flaps 54 are made of flexible material so that they are bent downwards to the direction of pocket 34, when solid waste is deposited in diaper 30. Flaps 54 are also made of smooth material so that the waste will slide off of them. Once the solid waste has passed through partition 50, flaps 54 return to their original position and serve as a cover over pocket 34 with the waste maintained in it.

Flexible partition 50 may be made of silicone, or any other material that is suitable for human use and that is flexible and smooth.

Partition 50 is removable, and is attached to leg cuffs 36a-b by Velcro ^{™} or any other suitable means of attachment. Partition 50 may be removed from diaper 30 after being used, and then can be cleaned and re-attached to a clean diaper.

Partition 50 is illustrated herein extending in a transverse direction, although it may be extended in a longitudinal direction.

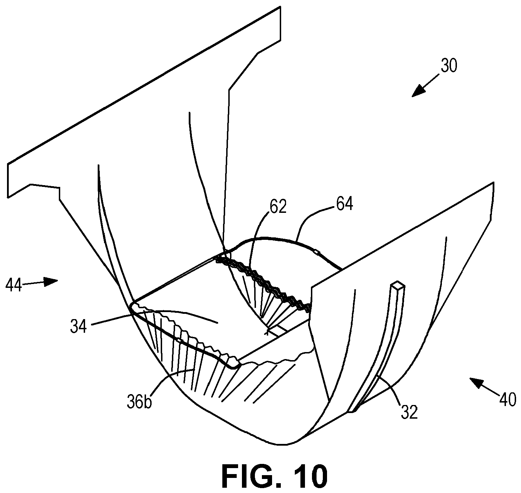

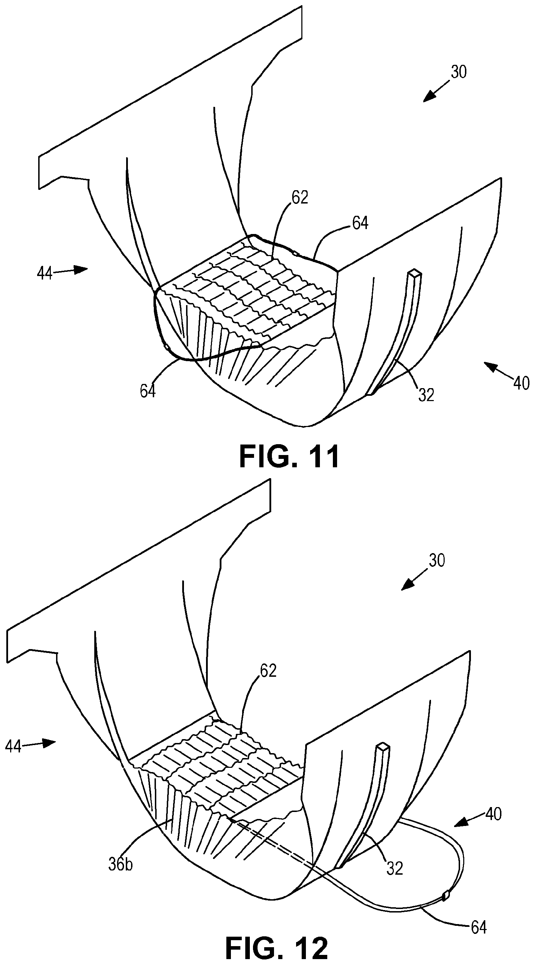

Referring now to Figs. 10-12 there is shown an additional embodiment of the present invention showing diaper 30 provided with a cover 62 for pocket 34 area. Cover 62 is attached to string 64 and by pulling a portion of string 64 which is located externally to diaper 30, cover 62 is spread over pocket 34 (shown in Fig. 11) so that the solid waste contained in pocket 34 does not touch the skin. Cover 62 may be folded by pulling string 64 on the opposite side to expose pocket 34 in the case of re-use by the diaper 30 wearer. Cover 62 may be spread and folded as needed.

Cover 62 may be spread and folded from either direction, from the front 40 to the rear 44 of diaper 30, or as shown in Fig. 12 from cuff 36a to cuff 36b.

This feature is especially suitable for the elderly, who can open and close the cover themselves, more than once if needed, taking into consideration the diaper's 30 capacity limit.

## Claims

1. An anti-irritant diaper (30) for containing solid waste excreted from a wearer and preventing it from contact with the wearer's skin, the diaper (30) having a front area (40), a middle area (42), a rear area (44), a pair of leg cuffs (36a, 36b) and closing tabs (38) and comprising:
a resilient flexible band (32) disposed longitudinally and centrally along the diaper (30);
a pocket (34) formed within said middle area (42) for receiving excreted solid waste and having a space above it, said space separating said pocket (34) from the wearer's skin; and
a one-directional flexible partition (50), situated above said pocket (34) and including a pair of dividers (52a, 52b), each of said dividers attached at one end to one of said pair of leg cuffs (36a, 36b) and segmented into a plurality of flexible flaps (54);
wherein said resilient flexible band (32) maintains the shape of said pocket (34), and
wherein said flaps (54) of said flexible partition (50) are configured to bend downward by the weight of solid waste, so as to allow it to enter into said pocket (34), and said flaps (54) are further configured to return thereafter to their original position, so as to prevent the waste from contacting the wearer's skin.

2. The anti-irritant diaper of claim 1, wherein said resilient flexible band (32) is disposed externally to the diaper (30) and is attached thereto by a plurality of tabs (33) disposed along the diaper (30) or is insertable into a designated external sleeve (35).

3. The anti-irritant diaper of claim 1, wherein said resilient flexible band (32) is insertable into a designated internal sleeve (45), formed in the diaper, so that said band (32) remains in place.

4. The anti-irritant diaper of claim 2 or 3, wherein said resilient flexible band (32) may be removed from the diaper (30) and inserted into another anti-irritant diaper.

5. The anti-irritant diaper of claim 1, wherein said flexible partition (50) serves as a cover over said pocket (34) so that any solid waste in said pocket (34) does not touch the wearer's skin.

6. The anti-irritant diaper of claim 1 wherein said flexible partition (50) may be removed from the diaper, cleaned and then re-attached to a clean diaper.

7. The anti-irritant diaper of claim 1, wherein said flaps (54) of said flexible partition (50), when in their original position, serve as a cover over said pocket (34).

## Patentansprüche

1. Reizlindernde Windel (30) zum Auffangen von festem Abfall, der von einem Träger ausgeschieden wird, und zum Verhindern, dass dieser mit der Haut des Trägers in Berührung kommt, wobei die Windel (30) einen vorderen Bereich (40), einen mittleren Bereich (42), einen hinteren Bereich (44), ein Paar Beinmanschetten (36a, 36b) und Verschlusslaschen (38) aufweist und Folgendes umfasst:
ein elastisches flexibles Band (32), das in Längsrichtung und zentral entlang der Windel (30) angeordnet ist;
eine Tasche (34), die innerhalb des mittleren Bereichs (42) zum Aufnehmen von ausgeschiedenem festen Abfall ausgebildet ist und einen Raum darüber aufweist, wobei der Raum die Tasche (34) von der Haut des Trägers trennt; und
eine unidirektionale flexible Teilung (50), die über der Tasche (34) gelegen ist und ein Paar Trennwände (52a, 52b) einschließt, wobei jede der Trennwände an einem Ende an einem des Paars von Beinmanschetten (36a, 36b) befestigt ist und in mehrere flexible Klappen (54) unterteilt ist;
wobei das elastische flexible Band (32) die Form der Tasche (34) beibehält, und
wobei die Klappen (54) der flexiblen Teilung (50) konfiguriert sind, um sich durch das Gewicht von festem Abfall nach unten zu biegen, um es ihm zu ermöglichen, in die Tasche (34) zu gelangen, und die Klappen (54) ferner konfiguriert sind, um danach in ihre ursprüngliche Position zurückzukehren, um zu verhindern, dass der Abfall die Haut des Trägers berührt.

2. Reizlindernde Windel nach Anspruch 1, wobei das elastische flexible Band (32) außerhalb der Windel (30) angeordnet ist und daran durch mehrere Laschen (33) befestigt ist, die entlang der Windel (30) angeordnet sind, oder in eine festgelegte äußere Hülse (35) einführbar ist.

3. Reizlindernde Windel nach Anspruch 1, wobei das elastische flexible Band (32) in eine festgelegte innere Hülse (45), die in der Windel ausgebildet ist, einführbar ist, so dass das Band (32) an Ort und Stelle bleibt.

4. Reizlindernde Windel nach Anspruch 2 oder 3, wobei das elastische flexible Band (32) von der Windel (30) entfernt und in eine andere reizlindernde Windel eingeführt werden kann.

5. Reizlindernde Windel nach Anspruch 1, wobei die flexible Teilung (50) als eine Abdeckung über der Tasche (34) dient, so dass beliebiger fester Abfall in der Tasche (34) die Haut des Trägers nicht berührt.

6. Reizlindernde Windel nach Anspruch 1, wobei die flexible Teilung (50) von der Windel entfernt, gereinigt und dann wieder an einer sauberen Windel befestigt werden kann.

7. Reizlindernde Windel nach Anspruch 1, wobei die Klappen (54) der flexiblen Teilung (50) in ihrer ursprünglichen Position als eine Abdeckung über der Tasche (34) dienen.

## Revendications

1. Couche anti-irritation (30) destinée à contenir les déchets solides excrétés par un utilisateur et à les empêcher d'entrer en contact avec la peau de l'utilisateur, la couche (30) ayant une zone avant (40), une zone médiane (42), une zone arrière (44), une paire de manchettes de jambe (36a, 36b) et des languettes de fermeture (38) et comprenant :
une bande flexible élastique (32) disposée longitudinalement et centralement le long de la couche (30) ;
une poche (34) formée à l'intérieur de ladite zone médiane (42) et destinée à recevoir des déchets solides excrétés et ayant un espace au-dessus de celle-ci, ledit espace séparant ladite poche (34) de la peau de l'utilisateur ; et
une cloison flexible (50) unidirectionnelle, située au-dessus de ladite poche (34) et comportant une paire de séparateurs (52a, 52b), chacun desdits séparateurs étant attaché à une extrémité de l'une de ladite paire de manchettes de jambe (36a, 36b) et segmenté en une pluralité de rabats (54) flexibles ;
ladite bande flexible élastique (32) maintenant la forme de ladite poche (34), et lesdits rabats (54) de ladite cloison flexible (50) étant conçus pour se plier vers le bas par le poids des déchets solides, de manière à leur permettre d'entrer dans ladite poche (34), et lesdits rabats (54) étant en outre conçus pour revenir par la suite à leur position d'origine, de manière à empêcher les déchets d'entrer en contact avec la peau de l'utilisateur.

2. Couche anti-irritation selon la revendication 1, dans laquelle ladite bande flexible élastique (32) est disposée à l'extérieur de la couche (30) et est attachée à celle-ci par une pluralité de languettes (33) disposées le long de la couche (30) ou peut être insérée dans un manchon externe désigné (35).

3. Couche anti-irritation selon la revendication 1, dans laquelle ladite bande flexible élastique (32) peut être insérée dans un manchon interne désigné (45), formé dans la couche, de sorte que ladite bande (32) reste en place.

4. Couche anti-irritation selon la revendication 2 ou 3, dans laquelle ladite bande flexible élastique (32) peut être retirée de la couche (30) et insérée dans une autre couche anti-irritation.

5. Couche anti-irritation selon la revendication 1, dans laquelle ladite cloison flexible (50) sert de couverture sur ladite poche (34) de sorte qu'aucun déchet solide dans ladite poche (34) ne touche la peau de l'utilisateur.

6. Couche anti-irritation selon la revendication 1, dans laquelle ladite cloison flexible (50) peut être retirée de la couche, nettoyée et ensuite ré-attachée à une couche propre.

7. Couche anti-irritation selon la revendication 1, dans laquelle lesdits rabats (54) de ladite cloison flexible (50), lorsqu'ils sont dans leur position d'origine, servent de couverture sur ladite poche (34).
